# EUROPEAN PATENT APPLICATION

(11) **EP 4 184 478 A1**
(43) Date of publication of application: **24.05.2023**
(21) Application number: 21841358.1
(22) Date of filing: 01.07.2021
(51) Int. Cl.: G08G 1/16, A61B 3/113, A61B 5/16, A61B 5/18

(54) **ELECTRONIC APPARATUS, INFORMATION PROCESSING DEVICE, ESTIMATION METHOD, AND ESTIMATION PROGRAM**

(30) Priority: 17.07.2020 JP 2020123230
(71) Applicant: Kyocera Corporation, Kyoto-shi Kyoto 612-8501 (JP)
(72) Inventor: SHINOZAKI Takayuki, Kyoto-shi, Kyoto 612-8501 (JP)
(74) Representative: Viering, Jentschura & Partner mbB Patent- und Rechtsanwälte
(86) International application number: PCT/JP2021/025038
(87) International publication number: WO 2022/014352

(57) **Abstract**

An electronic device 10 includes an image-capturing unit 11, a line-of-sight detector 12, and a controller 14. The image-capturing unit 11 generates an image corresponding to a view by performing image capturing. The line-of-sight detector detects a line of sight of a subject with respect to the view. The controller 14 estimates an alertness level of the subject based on an image and a line of sight. The controller 14 functions as an estimator. The estimator is constructed based on learning data obtained by machine learning a relationship between a learning image, a line of sight of a training subject with respect to the learning image, and biological information relating to an alertness level of the training subject.

## Description

### CROSS-REFERENCE TO RELATED APPLICATIONS

This application claims priority of Japanese Patent Application No. 2020-123230 filed in Japan on July 17, 2020 and the entire disclosure of this application is hereby incorporated by reference.

### TECHNICAL FIELD

The present disclosure relates to an electronic device, an information processing device, an estimating method, and an estimating program.

### BACKGROUND OF INVENTION

The attention of a driver is required for safe operation of a mobile object. Therefore, studies have been conducted on observing the driver's attention and issuing warnings to the driver or providing driving assistance when the driver's attention falls. As a way of observing attention, a method has been proposed in which cumulative visibility, which is a cumulative value of the degree of overlap of the line of sight with an object such as an oncoming vehicle around the driver's own vehicle, is calculated and compared with a reference value (refer to Patent Literature 1). An attention estimating system has also been proposed. The attention estimating system estimates whether or not a person being monitored visually recognizes an object to be viewed (refer to Patent Literature 2).

### CITATION LIST

### PATENT LITERATURE

Patent Literature 1: International Publication No. 2008-029802
Patent Literature 2: Japanese Unexamined Patent Application Publication No. 2015-207163

### SUMMARY

In order to solve the problem described above, in a First Aspect, an electronic device includes an image-capturing unit, a line-of-sight detector, and a controller.

The image-capturing unit is configured to generate an image corresponding to a view by performing image capturing.

The line-of-sight detector is configured to detect a line of sight of a subject with respect to the view.

The controller is configured to estimate an alertness level of the subject based on the image and the line of sight.

The controller functions as an estimator constructed based on learning data obtained by machine learning a relationship between a learning image, a line of sight of a training subject with respect to the learning image, and biological information relating to an alertness level of the training subject.

In a Second Aspect, an information processing device includes an acquiring unit, a controller, and an output unit.

The acquiring unit is configured to acquire an image corresponding to a view and a line of sight of a subject with respect to the view.

The controller is configured to estimate an alertness level of the subject based on the image and the line of sight.

The output unit is configured to output the alertness level.

The controller functions as an estimator constructed based on learning data obtained by machine learning a relationship between a learning image, a line of sight of a training subject with respect to the learning image, and biological information relating to an alertness level of the training subject.

In a Third Aspect, an estimating method includes: generating an image corresponding to a view by performing image capturing;
detecting a line of sight of a subject with respect to the view; and
estimating an alertness level of the subject based on the image and the line of sight.

The estimating estimates an alertness level based on learning data obtained by machine learning a relationship between a learning image, a line of sight of a training subject with respect to the learning image, and biological information relating to an alertness level of the training subject.

In a Fourth Aspect, an estimating program causes a computer to function as an image-capturing unit, a line-of-sight detector, and a controller.

The image-capturing unit is configured to generate an image corresponding to a view by performing image capturing.

The line-of-sight detector is configured to detect a line of sight of a subject with respect to the view.

The controller is configured to estimate an alertness level of the subject based on the image and the line of sight.

The controller functions as an estimator constructed based on learning data obtained by machine learning a relationship between a learning image, a line of sight of a training subject with respect to the learning image, and biological information relating to an alertness level of the training subject.

### BRIEF DESCRIPTION OF THE DRAWINGS

FIG. 1 is a block diagram illustrating the outline configuration of an electronic device according to an embodiment.
FIG. 2 is a diagram for describing the relationship between a line of sight detected by a line-of-sight detector and an image.
FIG. 3 is a flowchart for describing estimation processing executed by a controller in FIG. 1.
FIG. 4 is a block diagram illustrating an outline configuration of an information processing device according to an embodiment.

### DESCRIPTION OF EMBODIMENTS

Hereafter, an electronic device to which an embodiment of the present disclosure has been applied will be described while referring to the drawings. The following description also serves as a description of an information processing device, an estimating method, and an estimating program to which the present disclosure has been applied.

An electronic device according to an embodiment of the present disclosure is provided in a mobile object, for example. Such mobile objects may include, for example, vehicles, ships, and aircraft. Vehicles may include, for example, automobiles, industrial vehicles, rail vehicles, motorhomes, and fixed-wing aircraft that taxi along runways. Automobiles may include, for example, passenger cars, trucks, buses, motorcycles, and trolleybuses. Industrial vehicles may include, for example, industrial vehicles used in agriculture and construction. Industrial vehicles may include, for example, forklift trucks and golf carts. Industrial vehicles used in agriculture may include, for example, tractors, cultivators, transplanters, binders, combine harvesters, and lawn mowers. Industrial vehicles used in construction may include, for example, bulldozers, scrapers, excavators, crane trucks, dump trucks, and road rollers. Vehicles may include human-powered vehicles. Categories of vehicles are not limited to the examples described above. For example, automobiles may include industrial vehicles that can travel along roads. One vehicle may be included in multiple categories. Ships may include, for example, jet skis, boats, and tankers. Aircraft may include, for example, fixed-wing and rotary-wing aircraft.

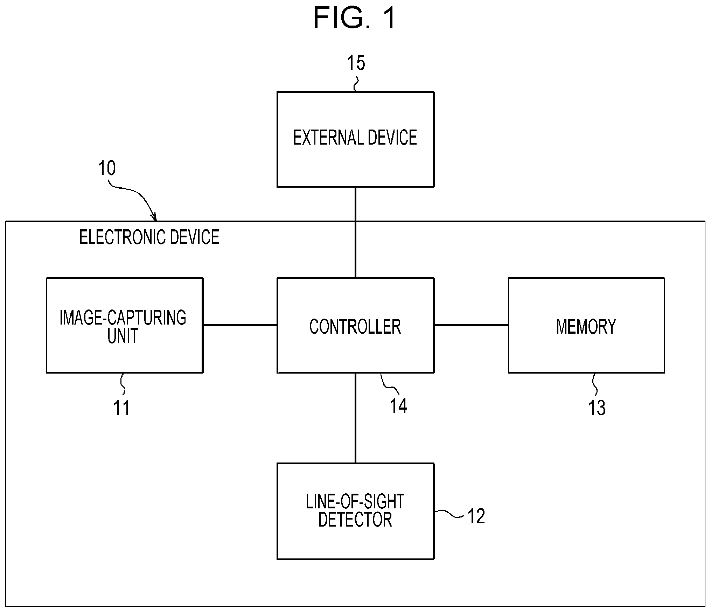

As illustrated in FIG. 1, an electronic device 10 according to an embodiment of the present disclosure includes an image-capturing unit 11, a line-of-sight detector 12, a memory 13, and a controller 14.

The image-capturing unit 11 is, for example, provided in a mobile object so as to be capable of capturing images of the view in the direction of movement of the mobile object. The image-capturing unit 11 is, for example, a camera capable of capturing images at a rate of 30 fps. The image-capturing unit 11 generates an image corresponding to the view by performing image capturing.

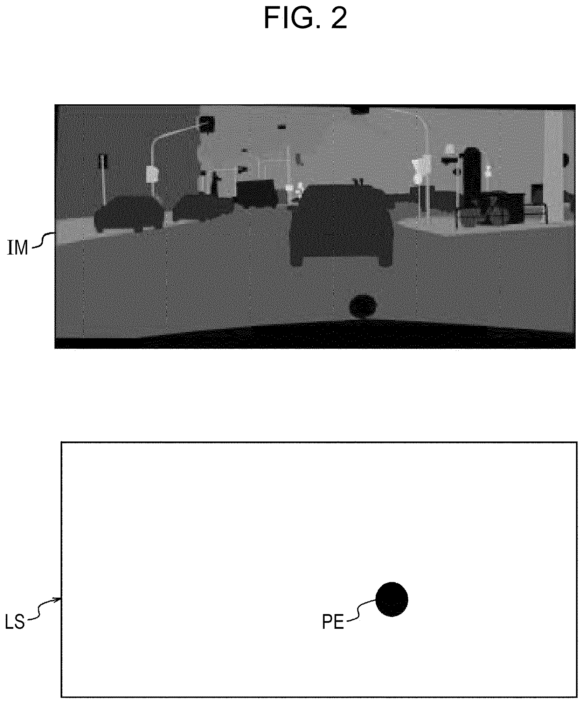

The line-of-sight detector 12 is, for example, provided in the mobile object so as to capable of detecting the line of sight of a subject sitting in the driver's seat of the mobile object. The line-of-sight detector 12 is, for example, either a contact-type eye tracker or a non-contact type eye tracker, and detects the line of sight of the subject with respect to the view. As illustrated in FIG. 2, a line of sight LS is, for example, illustrated as the direction of a line of sight corresponding to a position PE in the same coordinate system as an image IM generated by image capturing performed by the image-capturing unit 11.

The line-of-sight detector 12 may detect a time series of the line of sight as line-of-sight data. More specifically, the line-of-sight detector 12 may detect the position of the line of sight on the image every time interval and output a time series of the positions of the line of sight as the line-of-sight data. The line-of-sight detector 12 may detect the line of sight at a higher rate, i.e., at a higher frequency than the image-capturing unit 11, and may accumulate a time series of the detected positions of the line of sight, and output the accumulated positions as a path of the line of sight in an image.

The memory 13 includes any suitable storage device such as a random access memory (RAM) or a read only memory (ROM). The memory 13 stores various programs that allow the controller 14 to function and a variety of information used by the controller 14.

The controller 14 includes one or more processors and memories. Such processors may include general-purpose processors into which specific programs are loaded to perform specific functions, and dedicated processors dedicated to specific processing. Dedicated processors may include an application specific integrated circuit (ASIC). Processors may include programmable logic devices (PLDs). PLDs may include field-programmable gate arrays (FPGAs). The controller 14 may be either a system-on-a-chip (SoC) or a system in a package (SiP), in which one or more processors work together. The controller 14 controls operation of each component of the electronic device 10.

In normal operation, the controller 14 causes the image-capturing unit 11 to perform continuous image capturing at a rate of, for example, 30 fps and continuously acquires images IM as information. The controller 14 causes the line-of-sight detector 12 to detect the line of sight LS of the subject while the image-capturing unit 11 is performing image capturing and acquires the line of sight LS in a time period substantially coinciding with the time point of capture of the image IM. The meaning of "a time period substantially coinciding with a time point of capture of the image IM" may include a single time point of detection or may include multiple time points of detection during the period from a time point of the image capture immediately preceding capture of the most recent image IM to a time point of the most recent capture of an image IM. In the case of a single time point of detection of the line of sight, the time period substantially coinciding with a time point of capture of an image IM does not need to include the exact time point, and the time period may include the time point closest to the time point of capture of an image IM in line of sight detection performed in the same period as the image capturing. The controller 14 associates the image IM and the line of sight LS detected in a period substantially coinciding with the time point of capture of the image IM and stores the associated image IM and line of sight LS in the memory 13.

The controller 14 may perform image processing on the acquired image IM. As described later, in a configuration where training of the estimator is performed using images obtained by performing prescribed image processing on normal captured images IM such as semantic segmentation images or the like, the controller 14 performs the prescribed image processing on an acquired image IM and stores the image IM in association with the line of sight LS in the memory 13. A semantic segmentation image is an image in which a label or a category has been assigned to all pixels within the image.

The controller 14 estimates the alertness level of the subject based on an image IM and a line of sight LS, the line of sight LS being detected during a time period that substantially coincides with the time point of capture of the image IM. More specifically, the controller 14 functions as an estimator that estimates biological information relating to an alertness level of the subject based on the image IM and the line of sight LS. Biological information relating to an alertness level includes the alertness level itself and biological information that allows the alertness level to be calculated or estimated. Information that allows the alertness level to be calculated or estimated includes, for example, at least one out of heart rate, EEG, blink frequency, percentage of eyelid closure (PERCLOS), and so on. Therefore, the estimator of the controller 14 may estimate the alertness level based on an image IM and a line of sight LS, or alternatively, the estimator may estimate biological information with which the alertness level may be calculated or estimated and then calculate or estimate the alertness level based on the estimated biological information.

The estimator consists of, for example, a multilayer neural network. As described later, the estimator is constructed by performing machine learning.

For example, the estimator estimates the alertness level of the subject based on an image IM and a line of sight LS, the time point of capture of the image IM and the time point of detection of the line of sight LS substantially coinciding with each other.

Alternatively, for example, in a configuration in which the estimator is constructed by learning a relationship of biological information relating to the alertness level for the path of a line of sight with respect to a single learning image, the estimator estimates the alertness level of the subject based a single image IM and the path of a line of sight LS detected during a period substantially coinciding with a time point of capture of the single image IM.

However, as described below, in a configuration in which the estimator is constructed based on learning using a path that includes a portion for which the movement speed of a line of sight LS is less than a threshold within a single learning image, the estimator may estimate the alertness level of the subject by excluding a portion for which the movement speed of the line of sight LS is greater than or equal to the threshold out of the path of the line of sight LS. In other words, the estimator may exclude information obtained during a saccade. The reason for excluding such information is that a saccade is an action that takes place during the process of moving to the next gazing point, and the position of the line of sight during the saccade has no meaning as a point being looked at. The estimator may determine whether or not a saccade is occurring based on the speed of movement between positions of the line of sight.

As described below, in a configuration where the estimator is constructed based on learning using multiple continuously captured learning images, the estimator may estimate the alertness level of the subject based on the multiple continuously captured images IM and the line of sight LS for each of the multiple images IM, in other words, the lines of sight LS associated with the multiple images IM. The estimator, for example, may estimate the alertness level based on images IM of frames within a prescribed time interval backwards from the most recent image IM.

As described below, in a configuration where the estimator is constructed based on learning using learning images for which the movement speed of the line of sight is less than a threshold with respect to multiple continuously captured learning images, the estimator may estimate the alertness level of the subject by excluding combinations of lines of sight LS and images IM associated with the lines of sight LS for which the movement speed of the line of sight LS is greater than or equal to a threshold, from among multiple continuously captured images IM and the line of sights LS for the multiple images IM. More specifically, when the amount of movement of a line of sight LS at a particular detection time from the line of sight LS at the immediately previous detection time exceeds a threshold, the estimator may exclude the combination of the line of sight LS at the particular detection time and the image IM associated with the detection time of that line of sight LS. In other words, the controller 14 may exclude information obtained during a saccade. The reason for excluding such information is that a saccade is an action that takes place during the process of moving to the next gazing point, and the position of the line of sight during the saccade has no meaning as a point being looked at. The controller 14 may determine whether or not a saccade is occurring based on the speed of movement between positions of the line of sight.

The controller 14 outputs the calculated alertness level to an external device 15. The external device 15 is a device that performs a prescribed operation based on the alertness level. The external device 15 may be, for example, a warning device that alerts the subject based on the alertness level, a driving assistance device that assists the subject in driving the mobile object based on the alertness level, or a driving device that operates the mobile object based on the alertness level.

The estimator is constructed based on learning data obtained by machine learning the relationship between a learning image, an actual line of sight, and biological information relating to an alertness level using multiple sets of a learning image, the actual line of sight of a training subject with respect to a view corresponding to the learning image, and biological information relating to the alertness level of the training subject.

The estimator may be constructed based on learning data obtained by machine learning the relationship between a learning image, the path of an actual line of sight, and biological information relating to an alertness level using a single learning image, the path of an actual line of sight of a training subject with respect to a view corresponding to the single learning image, and biological information relating to the alertness level of the training subject. The estimator may further be constructed based on machine learning of a single training image, a portion of a line of sight within the single learning image for which the movement speed of the line of sight is less than a threshold, and biological information relating to each system.

The estimator may be further constructed based on machine learning of multiple continuously captured learning images, actual lines of sight of a training subject with respect to views corresponding to the multiple learning images, and biological information relating to the alertness level of the training subject at every time point when the multiple learning images are generated. The estimator may be further constructed based on machine learning of learning images, selected from among with respect to multiple continuously taken learning images, for which the speed of movement of the line of sight is less than a threshold, lines of sight, and biological information relating to an alertness level.

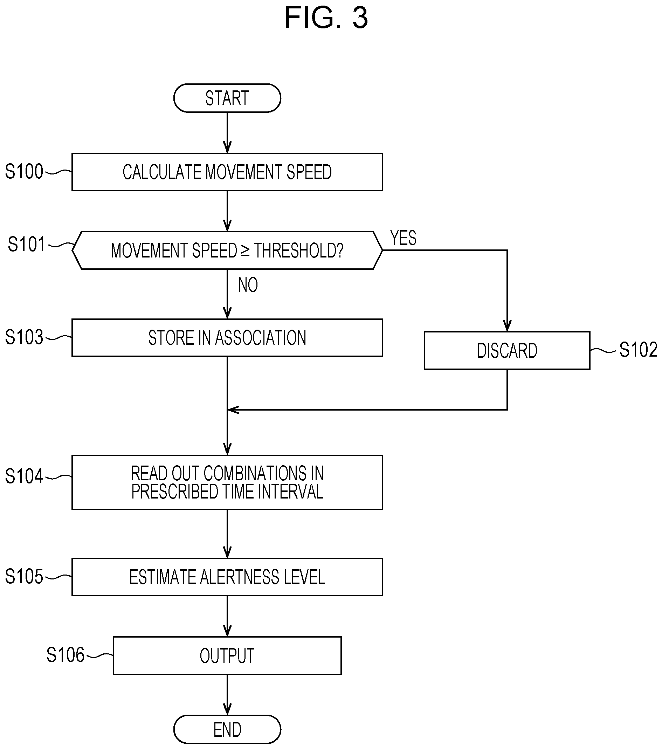

Next, estimation processing performed by the controller 14 in this embodiment will be described using the flowchart in FIG. 3. The estimation processing starts whenever the controller 14 acquires an image IM of one frame and a line of sight LS.

In Step S100, the controller 14 calculates the movement speed of the acquired line of sight LS based on a comparison of the acquired line of sight LS and a position PE of the line of sight LS acquired immediately previously. After the calculation, the process advances to Step S101.

In Step S101, the controller 14 determines whether the movement speed calculated in Step S101 is greater than or equal to a threshold. When the movement speed is greater than or equal to the threshold, the process advances to Step S102. When the movement speed is not greater than or equal to the threshold, the process advances to Step S103.

In Step S102, the controller 14 discards the image IM and the line of sight LS acquired at the start of the estimation processing. After the discarding, the process advances to Step S104.

In Step S103, the controller 14 stores the image IM and the line of sight LS acquired at the start of the estimation processing in the memory 13 in association with each other. After storing the data, the process advances to Step S104.

In Step S104, the controller 14 reads, from the memory 13, combinations of an image IM and a line of sight LS stored within a prescribed time interval backward. After that, the process advances to Step S105.

In Step S105, the controller 14 functions as an estimator in order to estimate the alertness level based on the multiple images IM and lines of sight LS included in the combinations read out in Step S104. After the estimation, the process advances to Step S 106.

In Step S106, the controller 14 outputs the alertness level estimated in Step S105 to the external device 15. After the output, the estimation processing ends.

The thus-configured electronic device 10 of this embodiment includes the controller 14. The controller 14 functions as an estimator. The estimator is constructed based on learning data obtained by machine learning the relationship between a learning image, the line of sight of a training subject with respect to the learning image, and biological information relating to the alertness level of the training subject. The electronic device 10 estimates the alertness level of a subject based on an image IM and a line of sight LS. For example, it is common for the object being looked at and the direction of looking to change for a variety of views, such as highways, urban areas, suburban areas, and residential areas. Therefore, it is difficult to improve the accuracy with which the alertness level is estimated by simply detecting only the movement of the line of sight LS. However, with the above-described configuration, since the electronic device 10 has already learned the objects that people look at in various views with different alertness levels, the electronic device 10 can estimate the alertness level with high accuracy based on an image IM and a line of sight LS corresponding to the view on that occasion. Since human attention is affected by the alertness level, the electronic device 10 can improve the accuracy with which the subject's attention is estimated in many different situations.

The electronic device 10 of this embodiment estimates the alertness level of the subject based on multiple continuously captured images IM and the lines of sight LS for the multiple images IM. With this configuration, since the electronic device 10 has already learned how the line of sight LS varies in various views depending on different alertness levels, the electronic device 10 can estimate the alertness level with even higher accuracy based on an image IM and a line of sight LS corresponding to the view on that occasion.

The electronic device 10 of this embodiment estimates the alertness level of the subject by excluding combinations of lines of sight LS and images IM, among the multiple continuously captured images IM and the line of sight LS for the multiple images IM, for which the speed of movement of the line of sight LS exceeds a threshold. The object that the subject is looking at in a view is expected to be a factor that significantly affects the alertness level. Therefore, it is expected that the line of sight LS will have little effect on the alertness level during a rapid change in line of sight from one object to another object, such as during a saccade. Therefore, since the electronic device 10 having the configuration described above excludes combinations of images IM and lines of sight LS that are considered to have little effect on the alertness level, the electronic device 10 can estimate the alertness level with even greater accuracy.

A variety of variations and amendments may be made to the content of the present disclosure based on the present disclosure by one skilled in the art. Therefore, it should be noted that such variations and amendments are included within the scope of the present disclosure. For example, in each embodiment, each functional part, each means, each step and so on can be added to other embodiments so long as there are no logical inconsistencies, or can be replaced with each functional part, each means, each step, and so on of other embodiments. In each embodiment, a plurality of each functional part, each means, each step, and so on can be combined into a single functional part, means, or step or divided into multiple functional parts, means, or steps. Each of the above-described embodiments of the present disclosure is not limited to faithful implementation of each of the described embodiments, and may be implemented by combining or omitting some of the features as appropriate.

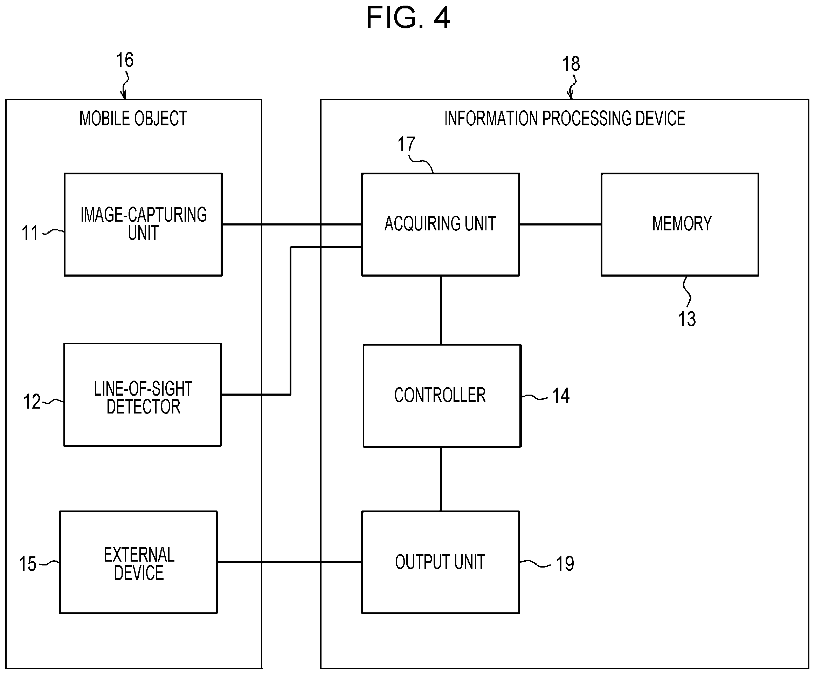

For example, in this embodiment, the electronic device 10 includes the image-capturing unit 11 and the line-of-sight detector 12, and the controller 14 acquires an image IM and a line of sight LS to use in estimating the alertness level, but this configuration does not have to be adopted. For example, the present disclosure may be realized as an information processing device to which a mobile object is connected via a network. An embodiment realized as an information processing device will be described while referring to FIG. 4. FIG. 4 is a block diagram illustrating an outline configuration of an information processing device 18 according to an embodiment. In this embodiment, the image-capturing unit 11 and the line-of-sight detector 12 are provided in a mobile object 16. The information processing device 18 acquires an image IM and a line of sight LS via an acquiring unit 17 by communicating with the mobile object 16. The controller 14 of the information processing device 18, which has the same configuration as the controller 14 of the electronic device 10 described above, estimates the alertness level based on the image IM and the line of sight LS. The information processing device 18 outputs the alertness level to the external device 15 of the mobile object 16 via an output unit 19.

Many aspects of the content of the present disclosure are presented as a series of operations executed by a computer system or other hardware capable of executing program instructions. Computer systems and other hardware include, for example, general-purpose computers, personal computers (PCs), dedicated computers, workstations, personal communications system (PCS), mobile (cellular) telephones, mobile telephones with data processing capabilities, RFID receivers, games consoles, electronic notepads, laptop computers, global positioning system (GPS) receivers or other programmable data processing devices. Note that in each embodiment, various operations are performed by dedicated circuits (for example, individual logic gates interconnected to perform specific functions) implemented using program instructions (software), or by logic blocks or program modules executed by one or more processors. Examples of "one or more processors that execute logic blocks or program modules" may include one or more microprocessors, a central processing unit (CPU), an application specific integrated circuit (ASIC), a digital signal processor (DSP), a programmable logic device (PLD), a field programmable gate array (FPGA), a processor, a controller, a microcontroller, a microprocessor, an electronic device, or another device designed to perform the functions described herein, and/or any combination of these. The embodiments described herein are implemented, for example, using hardware, software, firmware, middleware, microcode, or any combination of these. Instructions may be program code or code segments for performing the required tasks. The instructions can be stored in a machine-readable non-transitory storage medium or another medium. Code segments may represent any combination of procedures, functions, subprograms, programs, routines, subroutines, modules, software packages, classes or instructions, data structures or program statements. Code segments transmit and/or receive information, data arguments, variables or stored content from and/or to other code segments or hardware circuits, and in this way, connect to other code segments or hardware circuits.

Note that a system is disclosed herein as having various modules and/or units that perform specific functions. These modules and units are illustrated in a schematic manner in order to briefly illustrate their functionality and do not necessarily represent specific hardware and/or software. In that sense, these modules, units, and other components may be hardware and/or software implemented to substantially perform the specific functions described herein. The various functions of the different components may be any combination of hardware and/or software or hardware and/or software used in isolation, and can be used separately or in any combination. Thus, various aspects of the contents of the present disclosure can be implemented in numerous different ways, all of which are included within the scope of the present disclosure.

### REFERENCE SIGNS

- 10: electronic device
- 11: image-capturing unit
- 12: line-of-sight detector
- 13: memory
- 14: controller
- 15: external device
- 16: mobile object
- 17: acquiring unit
- 18: information processing device
- 19: output unit
- IM: image
- LS: line of sight
- PE: position corresponding to direction of line of sight

## Claims

1. An electronic device comprising:
an image-capturing unit configured to generate an image corresponding to a view by performing image capturing;
a line-of-sight detector configured to detect a line of sight of a subject with respect to the view; and
a controller configured to estimate an alertness level of the subject based on the image and the line of sight,
wherein the controller functions as an estimator constructed based on learning data obtained by machine learning a relationship between a learning image, a line of sight of a training subject with respect to the learning image, and biological information relating to an alertness level of the training subject.

2. The electronic device according to claim 1,
wherein the controller estimates an alertness level of the subject based on multiple continuously captured images and the lines of sight for the multiple images.

3. The electronic device according to claim 2,
wherein the controller estimates the alertness level of the subject by excluding combinations of lines of sight and images for which a speed of movement of the line of sight exceeds a threshold among the multiple continuously captured images and the lines of sight for the images.

4. An information processing device comprising:
an acquiring unit configured to acquire an image corresponding to a view and a line of sight of a subject with respect to the view;
a controller configured to estimate an alertness level of the subject based on the image and the line of sight; and
an output unit configured to output the alertness level,
wherein the controller functions as an estimator constructed based on learning data obtained by machine learning a relationship between a learning image, a line of sight of a training subject with respect to the learning image, and biological information relating to an alertness level of the training subject.

5. An estimating method comprising:
generating an image corresponding to a view by performing image capturing;
detecting a line of sight of a subject with respect to the view; and
estimating an alertness level of the subject based on the image and the line of sight,
wherein the estimating estimates an alertness level based on learning data obtained by machine learning a relationship between a learning image, a line of sight of a training subject with respect to the learning image, and biological information relating to an alertness level of the training subject.

6. An estimating program configured to cause a computer to function as:
an image-capturing unit configured to generate an image corresponding to a view by performing image capturing;
a line-of-sight detector configured to detect a line of sight of a subject with respect to the view; and
a controller configured to estimate an alertness level of the subject based on the image and the line of sight,
wherein the controller functions as an estimator constructed based on learning data obtained by machine learning a relationship between a learning image, a line of sight of a training subject with respect to the learning image, and biological information relating to an alertness level of the training subject.
